# EUROPEAN PATENT APPLICATION

(11) **EP 3 939 967 A1**
(43) Date of publication of application: **19.01.2022**
(21) Application number: 20186015.2
(22) Date of filing: 15.07.2020
(51) Int. Cl.: C07D 257/04, C07C 253/30, C07C 255/58

(54) **A CONTINUOUS PROCESS FOR THE PREPARATION OF (S)-METHYL N-((2'-CYANO-[1,1'-BIPHENYL]-4-YL)METHYL)-N-PENTANOYLVALINATE IN A FLOW REACTOR**

(71) Applicant: KRKA, d.d., Novo mesto, 8000 Novo mesto (SI)
(72) Inventor: Ruzic, Milo, 3000 Celje (SI); Svetelj, Jure, 1000 Ljubljana (SI); Zupancic, Silvo, 8000 Novo mesto (SI); Plazl, Igor, 1000 Ljubljana (SI); Znidarsic Plazl, Polona, 1000 Ljubljana (SI); Bitenc, Marko, 8501 Novo mesto (SI); Regina, Blaz, 8000 Novo mesto (SI)
(74) Representative: Hoefer & Partner Patentanwälte mbB

(57) **Abstract**

The present invention relates to a continuous process for the preparation of valsartan precursor (S)-methyl N-((2'-cyano-[1,1'-biphenyl]-4-yl)methyl)-N-pentanoylvalinate from (S)-methyl ((2'-cyano-[1,1'-biphenyl]-4-yl)methyl)valinate hydrochloride by N-acylation with valeryl chloride carried out in a flow reactor.

## Description

### Field of the invention

The present invention relates to a continuous process for the preparation of valsartan precursor (S)-methyl N-((2'-cyano-[1,1'-biphenyl]-4-yl)methyl)-N-pentanoylvalinate (VSN) from (S)-methyl ((2'-cyano-[1,1'-biphenyl]-4-yl)methyl)valinate hydrochloride (VSV) by N-acylation with valeryl chloride carried out in a flow reactor.

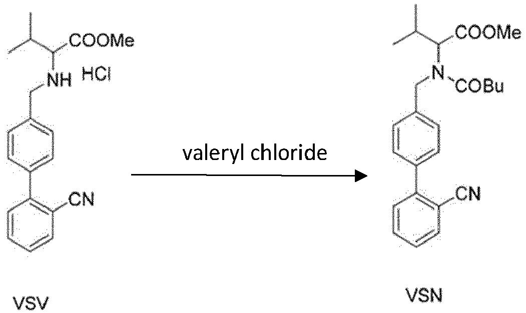

### Background of the invention

Valsartan is a non-peptide, orally active, specific angiotensin II antagonist, useful in the treatment of hypertension.

Valsartan, its pharmaceutically acceptable salts, pharmaceutical compositions comprising valsartan and their use in treating high blood pressure and cardiac insufficiency are disclosed in US 5,399,578.

According to CN 1317485 VSN is prepared by the acylation of VSV with valeryl chloride in an aromatic solvent in the presence of 4-dimethylaminopyridine and carbonate as catalysts. Similar process is described in WO 2005/49587, wherein VSN is prepared by the acylation of VSV with valeryl chloride in dichloromethane in the presence of diisopropyl ethyl amine and carbonate. In WO 2012/001484 VSN is prepared by reacting VSV with valeryl chloride in the presence of a heterocyclic amine base.

Use of organic amines in a process for preparing VSV might lead to a formation of carcinogenic nitrosoamines. Several recalls of valsartan products contaminated with nitrosoamines have happened recently.

IP. Com Journal (2006), No. IPCOM000138267D and WO 2010/91169 disclose processes for the acylation of VSV with valeryl chloride in the presence of carbonate in a mixture of water and xylene to produce VSN. Similar processes are described in CN 103923028, US 2009/203920 and WO2012/148148, wherein a mixture of water and toluene is used as solvent in acylation step. According to US 7,659,406 VSN may be produced by the acylation of VSV with valeryl chloride in the presence of an inorganic base, such as hydroxides and carbonates, in various solvents. CN110078640 discloses a process for preparing VSN by the acylation of VSV with valeryl chloride in the presence of carbonate and sodium chloride or potassium chloride.

Use of inorganic bases, particularly carbonates, requires biphase solvent system comprising an organic solvent and water. Furthermore, use of inorganic bases is faced with several issues, such as a presence of solid intermediates in a reaction mixture and a formation of solid by products, such as sodium hydrogencarbonate and sodium chloride. These issues have negative impacts on efficiency of a process.

Hence, there is a need for an effective process for the preparation of valsartan precursor VSN.

Some continuous processes for preparing some valsartan precursors carried out in flow reactors have been reported already: Pandarus V. et al. (2014). Org. Process. Res. Dev. 18:1550-1555; Nagaki A. et al. (2016). Org. Process. Res. Dev. 20:687-691; Hiebler, K. et al. (2019). J. Flow. Chem.

However, a process for preparing VSN has not been performed in a flow reactor yet. Because it is well known that processes wherein solid substances are present at any stage of the process, e.g. solid reactants, intermediates, products, or by-products, are not appropriate for being performed in flow reactors.

Surprisingly, we have found that a process for preparing VSN by N-acylation of VSV with valeryl chloride can be efficiently carried out continuously in a flow reactor.

### Summary of the invention

The present invention in particular provides various aspects, advantageous features and preferred embodiments as summarized in the following items, which respectively alone or in combination particularly contribute to solving the object of the invention and eventually provide additional advantages:
(1) A process for the preparation of VSN by acylation of VSV with valeryl chloride comprising:
   i) contacting, preferably continuously mixing, in a flow reactor a solution of VSV and a solution of valeryl chloride in the presence of a carbonate, thereby producing VSN; and
   ii) continuously removing a product stream comprising VSN from said flow reactor, preferably after performing the continuous extraction *in situ,* and then subjecting the product stream comprising VSN to a separation of organic and water phases, preferably via continuous flow separator.
(2) The process according to item (1), wherein the carbonate is selected from the group consisting of sodium carbonate, sodium hydrogen carbonate, potassium carbonate, potassium hydrogen carbonate, and mixtures thereof, preferably the carbonate is sodium carbonate.
(3) The process according to item (1) or item (2), wherein a molar ratio between VSV and the carbonate in the flow reactor is from 1:1 to 1:10, preferably from 1:1 to 1:6, most preferably from 1:2 to 1:4.
(4) The process according to any one of items (1) to (3), wherein a molar ratio between VSV and valeryl chloride in the flow reactor is from 1:10 to 10:1, preferably from 1:6 to 8:1, more preferably from 1:3 to 6:1, most preferably from 1:2 to 4:1.
(5) The process according to any one of items (1) to (4), wherein a solvent for preparing a solution of VSV is water, an aromatic hydrocarbon solvent or a mixture thereof.
(6) The process according to item (5), wherein the aromatic hydrocarbon solvent is selected from the group consisting of toluene, xylene, benzene, ethylbenzene and mixtures thereof, more preferably toluene or xylene, most preferably toluene.
(7) The process according to any one of items (1) to (6), wherein a solvent for preparing a solution of valeryl chloride is an aromatic hydrocarbon solvent, preferably selected from the group consisting of toluene, xylene, benzene, ethylbenzene and mixtures thereof, more preferably toluene or xylene, most preferably toluene.
(8) The process according to any one of items (1) to (7), wherein the same aromatic hydrocarbon solvent is used for preparing the solution of VSV and the solution of valeryl chloride, respectively.
(9) The process according to any one of items (1) to (8), wherein a solvent for preparing a solution of the carbonate is water.
(10) The process according to any one of items (1) to (9), wherein a volume ratio between the aromatic hydrocarbon solvent and water in the flow reactor is from 5:1 to 1:5, preferably from 3:1 to 1:3, more preferably from 2:1 to 1:2, most preferably from 3:2 to 1:1.
(11) The process according to any one of items (1) to (10), wherein a concentration of VSV in the flow reactor is from 0.05 mmol/L to 1 mmol/mL, preferably from 0.05 mmol/mL to 0.5 mmol/mL, more preferably from 0.05 mmol/mL to 0.43 mmol/mL, most preferably from 0.10 mmol/mL to 0.25 mmol/mL.
(12) The process according to any one of items (1) to (11), wherein a concentration of the carbonate in the flow reactor is from 0.05 mmol/L to 5.0 mmol/mL, preferably from 0.05 mmol/mL to 3.0 mmol/mL, more preferably from 0.1 mmol/mL to 2.0 mmol/mL, most preferably from 0.1 mmol/mL to 1.0 mmol/mL.
(13) The process according to any one of items (1) to (12), wherein a concentration of valeryl chloride in the flow reactor is from 0.05 mmol/L to 5.0 mmol/mL, preferably from 0.05 mmol/mL to 3.0 mmol/mL, more preferably from 0.1 mmol/mL to 2.0 mmol/mL, most preferably from 0.1 mmol/mL to 1.0 mmol/mL.
(14) The process according to any one of items (1) to (13), wherein a temperature in the flow reactor is at least 20°C, more preferably at least 40°C, even more preferably at least 45°C, even more preferably a temperature in a flow reactor is between 20°C and 80°C, even more preferably between 40°C and 70°C, even more preferably between 40°C and 60°C, even more preferably between 40°C and 55°C, most preferably between 40°C and 50°C.
(15) A process for the preparation of VSN by acylation of VSV with valeryl chloride comprising:
   i) providing a first stream containing a solution of VSV and a carbonate;
   ii) providing a second stream containing a solution of valeryl chloride;
   iii) contacting, preferably continuously mixing, said streams in a flow reactor, thereby producing VSN; and
   iv) continuously removing a product stream from said flow reactor, preferably after performing the continuous extraction *in situ,* and then subjecting the product stream comprising VSN to a separation of organic and water phases, preferably via continuous flow separator.
(16) The process according to item (15), wherein the carbonate is selected from the group consisting of sodium carbonate, sodium hydrogen carbonate, potassium carbonate, potassium hydrogen carbonate and mixtures thereof, preferably the carbonate is sodium carbonate.
(17) The process according to item (15) or item (16), wherein a solvent for preparing the solution of VSV and the carbonate is water or a mixture of water and an aromatic hydrocarbon solvent.
(18) The process according to item (17), wherein the aromatic hydrocarbon solvent is selected from the group consisting of toluene, xylene, benzene, ethylbenzene and mixtures thereof, more preferably toluene or xylene, most preferably toluene.
(19) The process according to item (17) or item (18), wherein the aromatic hydrocarbon solvent and water are mixed in a volume ratio from 2:1 to 1:5, preferably from 1:1 to 1:2, most preferably 2:3.
(20) The process according to any one of items (15) to (19), wherein a molar ratio between VSV and the carbonate in the solution of VSV and a carbonate is from 1:1 to 1:10, preferably from 1:1 to 1:6, most preferably from 1:2 to 1:4.
(21) The process according to any one of items (15) to (20), wherein a concentration of VSV in the solution of VSV and the carbonate is from 0.05 mmol/L to 1 mmol/mL, preferably from 0.1 mmol/mL to 0.5 mmol/mL, more preferably from 0.15 mmol/mL to 0.3 mmol/mL, most preferably from 0.20 mmol/mL to 0.25 mmol/mL.
(22) The process according to any one of items (15) to (21), wherein a concentration of the carbonate in the solution of VSV and the carbonate is from 0.1 mmol/L to 5.0 mmol/mL, preferably from 0.3 mmol/mL to 3.0 mmol/mL, more preferably from 0.4 mmol/mL to 2.0 mmol/mL, most preferably from 0.5 mmol/mL to 1.0 mmol/mL.
(23) The process according to any one of items (15) to (22), wherein a solvent for preparing the solution of valeryl chloride is an aromatic hydrocarbon solvent, preferably selected from the group consisting of toluene, xylene, benzene, ethylbenzene and mixtures thereof, more preferably toluene or xylene, most preferably toluene.
(24) The process according to any one of items (15) to (23), wherein the same aromatic hydrocarbon solvent is used for preparing the solution of VSV and the solution of valeryl chloride, respectively.
(25) The process according to any one of items (15) to (24), wherein a concentration of valeryl chloride in the solution of valeryl chloride is from 0.1 mmol/mL to 5.0 mmol/mL, preferably from 0.5 mmol/mL to 4.0 mmol/mL, more preferably from 0.8 mmol/mL to 2.5 mmol/mL, most preferably from 1.0 mmol/mL to 2.0 mmol/mL.
(26) The process according to any one of items (15) to (25), wherein a molar ratio between VSV and valeryl chloride in the flow reactor is from 1:10 to 10:1, preferably from 1:6 to 8:1, more preferably from 1:4 to 6:1, even more preferably from 1:2 to 4:1.
(27) The process according to any one of items (15) to (26), wherein a concentration of VSV in the flow reactor is from 0.05 mmol/L to 1 mmol/mL, preferably from 0.05 mmol/mL to 0.5 mmol/mL, more preferably from 0.05 mmol/mL to 0.43 mmol/mL, most preferably from 0.10 mmol/mL to 0.25 mmol/mL.
(28) The process according to any one of items (15) to (27), wherein a concentration of the carbonate in the flow reactor is from 0.05 mmol/L to 5.0 mmol/mL, preferably from 0.05 mmol/mL to 3.0 mmol/mL, more preferably from 0.1 mmol/mL to 2.0 mmol/mL, most preferably from 0.1 mmol/mL to 1.0 mmol/mL.
(29) The process according to any one of items (15) to (28), wherein a concentration of valeryl chloride in the flow reactor is from 0.05 mmol/L to 5.0 mmol/mL, preferably from 0.05 mmol/mL to 3.0 mmol/mL, more preferably from 0.1 mmol/mL to 2.0 mmol/mL, most preferably from 0.1 mmol/mL to 1.0 mmol/mL.
(30) The process according to any one of items (15) to (29), wherein a volume ratio between aromatic hydrocarbon solvent and water in the flow reactor is from 5:1 to 1:5, preferably from 3:1 to 1:3, more preferably from 2:1 to 1:2, most preferably from 3:2 to 1:1.
(31) The process according to any one of items (15) to (30), wherein the first stream containing the solution of VSV and the carbonate is added into the flow reactor through the first addition port and the second stream containing a solution of valeryl chloride is added through the second addition port.
(32) The process according to any one of items (15) to (31), wherein the first stream containing the solution of VSV and the carbonate and the second stream containing a solution of valeryl chloride are added into the flow reactor simultaneously.
(33) The process according to any one of items (15) to (31), wherein the first stream containing the solution of VSV and the carbonate and the second stream containing a solution of valeryl chloride are added into the flow reactor in successive steps.
(34) The process according to any one of items (15) to (33), wherein the second stream containing the solution of valeryl chloride is added in a flow reactor in one portion or in more portions.
(35) The process according to any one of items (15) to (34), wherein the second stream containing the solution of valeryl chloride is added in a flow reactor in one portion.
(36) The process according to any one of items (15) to (34), wherein the second stream containing the solution of valeryl chloride is added in a flow reactor in two to five portions, preferably in two to four portions.
(37) The process according to item (35), wherein a molar ratio between VSV and valeryl chloride in the flow reactor is from 1:1 to 1:5, preferably from 1:1 to 1:4, more preferably from 1:1 to 1:3.
(38) The process according to item (36), wherein a molar ratio between VSV and valeryl chloride in a flow reactor in each individual addition step is from 1:1 to 10:1, preferably from 2:1 to 6:1, more preferably from 2:1 to 5:1.
(39) The process according to any one of items (15) to (38), wherein a temperature in the flow reactor is at least 20°C, more preferably at least 40°C, even more preferably at least 45°C, even more preferably a temperature in a flow reactor is between 20°C and 80°C, even more preferably between 40°C and 70°C, even more preferably between 40°C and 60°C, even more preferably between 40°C and 55°C, most preferably between 40°C and 50°C.
(40) A process for the preparation of VSN by acylation of VSV with valeryl chloride comprising:
   i) providing a first stream containing a solution of VSV;
   ii) providing a second stream containing a solution of carbonate;
   iii) providing a third stream containing a solution of valeryl chloride;
   iv) contacting, preferably continuously mixing, said streams in a flow reactor, thereby producing VSN; and
   v) continuously removing a product stream comprising VSN from said flow reactor, preferably after performing the continuous extraction *in situ,* and then subjecting the product stream comprising VSN to a separation of organic and water phases, preferably via continuous flow separator.
(41) The process according to item (40), wherein the carbonate is selected from the group consisting of sodium carbonate, sodium hydrogen carbonate, potassium carbonate, potassium hydrogen carbonate and mixtures thereof, preferably the carbonate is sodium carbonate.
(42) The process according to item (40) or item (41), wherein a solvent for preparing a solution of VSV is water or a mixture of water and an aromatic hydrocarbon solvent.
(43) The process according to item (42), wherein the aromatic hydrocarbon solvent is selected from the group consisting of toluene, xylene, benzene, ethylbenzene and mixtures thereof, more preferably toluene or xylene, most preferably toluene.
(44) The process according to item (42) or item (43), wherein the aromatic hydrocarbon solvent and water are mixed in a volume ratio from 2:1 to 1:5, preferably from 1:1 to 1:2, most preferably 2:3.
(45) The process according to any one of items (40) to (44), wherein a concentration of VSV in the solution of VSV is from 0.05 mmol/L to 1 mmol/mL, preferably from 0.1 mmol/mL to 0.5 mmol/mL, more preferably from 0.15 mmol/mL to 0.3 mmol/mL, most preferably from 0.20 mmol/mL to 0.25 mmol/mL.
(46) The process according to any one of items (40) to (45), wherein a solvent for preparing the solution of carbonate is water.
(47) The process according to any one of items (40) to (46), wherein a concentration of the carbonate in the solution of the carbonate is from 0.1 mmol/L to 5.0 mmol/mL, preferably from 0.3 mmol/mL to 3.0 mmol/mL, more preferably from 0.4 mmol/mL to 2.0 mmol/mL, most preferably from 0.5 mmol/mL to 1.0 mmol/mL.
(48) The process according to any one of items (40) to (47), wherein a solvent for preparing the solution of valeryl chloride is an aromatic hydrocarbon solvent, preferably selected from group consisting of toluene, xylene, benzene, ethylbenzene and mixtures thereof, more preferably toluene or xylene, most preferably toluene.
(49) The process according to any one of items (40) to (48), wherein the same aromatic hydrocarbon solvent is used for preparing the solution of VSV and the solution of valeryl chloride, respectively.
(50) The process according to any one of items (40) to (49), wherein a concentration of valeryl chloride in the solution of valeryl chloride is from 0.1 mmol/mL to 5.0 mmol/mL, preferably from 0.5 mmol/mL to 4.0 mmol/mL, more preferably from 0.8 mmol/mL to 2.5 mmol/mL, most preferably from 1.0 mmol/mL to 2.0 mmol/mL.
(51) The process according to any one of items (40) to (50), wherein a molar ratio between VSV and the carbonate in the flow reactor is from 1:1 to 1:10, preferably from 1:1 to 1:6, most preferably from 1:2 to 1:4.
(52) The process according to any one of items (40) to (51), wherein a molar ratio between VSV and valeryl chloride in the flow reactor is from 1:10 to 10:1, preferably from 1:6 to 8:1, more preferably from 1:3 to 6:1, even more preferably from 1:2 to 4:1.
(52) The process according to any one of items (40) to (51), wherein a concentration of VSV in the flow reactor is from 0.05 mmol/L to 1 mmol/mL, preferably from 0.05 mmol/mL to 0.5 mmol/mL, more preferably from 0.05 mmol/mL to 0.43 mmol/mL, most preferably from 0.10 mmol/mL to 0.25 mmol/mL.
(53) The process according to any one of items (40) to (52), wherein a concentration of the carbonate in the flow reactor is from 0.05 mmol/L to 5.0 mmol/mL, preferably from 0.05 mmol/mL to 3.0 mmol/mL, more preferably from 0.1 mmol/mL to 2.0 mmol/mL, most preferably from 0.1 mmol/mL to 1.0 mmol/mL.
(54) The process according to any one of items (40) to (53), wherein a concentration of valeryl chloride in the flow reactor is from 0.05 mmol/L to 5.0 mmol/mL, preferably from 0.05 mmol/mL to 3.0 mmol/mL, more preferably from 0.1 mmol/mL to 2.0 mmol/mL, most preferably from 0.1 mmol/mL to 1.0 mmol/mL.
(55) The process according to any one of items (40) to (54), wherein a volume ratio between the aromatic hydrocarbon solvent and water in the flow reactor is from 5:1 to 1:5, preferably from 3:1 to 1:3, more preferably from 2:1 to 1:2, most preferably from 3:2 to 1:1.
(56) The process according to any one of items (40) to (55), wherein the first stream containing the solution of VSV is added into the flow reactor through the first addition port, the second stream containing the solution of the carbonate is added through the second addition port and the third stream containing the solution of valeryl chloride is added through the third addition port.
(57) The process according to any one of items (40) to (56), wherein the first stream containing the solution of VSV, the second stream containing the solution of the carbonate and the third stream containing the solution of valeryl chloride are added into the flow reactor simultaneously.
(58) The process according to any one of items (40) to (56), wherein the first stream containing the solution of VSV, the second stream containing the solution of the carbonate and the third stream containing the solution of valeryl chloride are added into the flow reactor in successive steps.
(59) The process according to any one of items (40) to (56), wherein the first stream containing the solution of VSV and the second stream containing the solution of the carbonate are added into the flow reactor simultaneously and the third stream containing the solution of valeryl chloride is added into the flow reactor in a successive step.
(60) The process according to any one of items (40) to (59), wherein the third stream containing the solution of valeryl chloride is added into the flow reactor in one portion or in more portions.
(61) The process according to any one of items (40) to (60), wherein the third stream containing the solution of valeryl chloride is added into the flow reactor in one portion.
(62) The process according to any one of items (40) to (60), wherein the third stream containing the solution of valeryl chloride is added into the flow reactor in two to five portions, preferably in two to four portions.
(63) The process according to item (61), wherein a molar ratio between VSV and valeryl chloride in the flow reactor is from 1:1 to 1:5, preferably from 1:1 to 1:4, more preferably from 1:1 to 1:3.
(64) The process according to item (62), wherein a molar ratio between VSV and valeryl chloride in the flow reactor in each individual step is from 1:1 to 10:1, preferably from 2:1 to 6:1, more preferably from 2:1 to 5:1.
(65) The process according to any one of items (40) to (64), wherein a temperature in the flow reactor is at least 20°C, more preferably at least 40°C, even more preferably at least 45°C, even more preferably a temperature in a flow reactor is between 20°C and 80°C, even more preferably between 40°C and 70°C, even more preferably between 40°C and 60°C, even more preferably between 40°C and 55°C, most preferably between 40°C and 50°C.
(66) The process according to any one of items (1) to (65), wherein the continuous flow separator is a membrane separator.
(67) The process according to any one of items (1) to (66), wherein a conversion rate of VSV to VSN is at least 95%, preferably at least 97%, more preferably at least 98%, even more preferably at least 99%, most preferably at least 99.5%.
(68) The process according to any one of items (1) to (67), wherein a purity of obtained VSN measured by HPLC is at least 95%, preferably at least 97%, more preferably at least 98% even more preferably at least 99%, most preferably at least 99.5%.
(69) The process according to any one of items (1) to (68) comprising further step(s) of converting obtained VSN to valsartan.
(70) The process according to item (69) comprising the following further steps:
   i) treating VSN with azide, preferably tributyltin azide or a mixture of NaN₃ and ZnCl₂, to form tetrazole intermediate,
   ii) hydrolysis of tetrazole intermediate obtained in step i), preferably with hydroxide or carbonate,
   iii) recovering valsartan.
(71) A process for the preparation of valsartan including the process for the preparation of VSN according to any one of items (1) to (68).
(72) A process for the preparation of valsartan comprising the following steps:
   i) preparing VSN according to any one of items (1) to (68),
   ii) treating VSN with azide, preferably tributyltin azide or a mixture of NaN₃ and ZnCl₂, to form tetrazole intermediate,
   iii) hydrolysis of tetrazole intermediate obtained in step ii), preferably with hydroxide or carbonate,
   iv) recovering valsartan.
(73) A process for the preparation of valsartan according to item (72) comprising the following steps:
   i) preparing VSN according to any one of items (1) to (14),
   ii) treating VSN with azide, preferably tributyltin azide or a mixture of NaN₃ and ZnCl₂, to form tetrazole intermediate,
   iii) hydrolysis of tetrazole intermediate obtained in step ii), preferably with hydroxide or carbonate,
   iv) recovering valsartan.
(74) A process for the preparation of valsartan according to item (71) comprising the following steps:
   i) preparing VSN according to any one of items (15) to (39),
   ii) treating VSN with azide, preferably tributyltin azide or a mixture of NaN₃ and ZnCl₂, to form tetrazole intermediate,
   iii) hydrolysis of tetrazole intermediate obtained in step ii), preferably with hydroxide or carbonate,
   iv) recovering valsartan.
(75) A process for the preparation of valsartan according to item (71) comprising the following steps:
   i) preparing VSN according to any one of items (40) to (65),
   ii) treating VSN with azide, preferably tributyltin azide or a mixture of NaN₃ and ZnCl₂, to form tetrazole intermediate,
   iii) hydrolysis of tetrazole intermediate obtained in step ii), preferably with hydroxide or carbonate,
   iv) recovering valsartan.

### Detailed description

In the following, the present invention is described in more details while referring to preferred embodiments and examples, which are presented however for illustrative purposes and shall not be construed to limit the invention in any way.

The term "VSN" means the compound (S)-methyl 2-(N-((2'-cyano-[1,1'-biphenyl]-4-yl)methyl)pentanamido)-3-methylbutanoate.
The term "VSV" means the compound (S)-methyl 2-(((2'-cyano-[1,1'-biphenyl]-4-yl)methyl)amino)-3-methylbutanoate hydrochloride (VSV)

First aspect of the present invention is a process for the preparation of (S)-methyl 2-(N-((2'-cyano-[1,1'-biphenyl]-4-yl)methyl)pentanamido)-3-methylbutanoate (VSN) by acylation of (S)-methyl 2-(((2'-cyano-[1,1'-biphenyl]-4-yl)methyl)amino)-3-methylbutanoate hydrochloride (VSV) with valeryl chloride comprising:
i) contacting, preferably continuously mixing, in a flow reactor a solution of VSV and a solution of valeryl chloride in the presence of a carbonate, thereby producing VSN; and
ii) continuously removing a product stream comprising VSN from said flow reactor, preferably after performing the continuous extraction *in situ,* and then subjecting the product stream comprising VSN to a separation of organic and water phases, preferably via continuous flow separator.

In particular, the process of the present invention can be a process for the preparation of VSN by acylation of VSV with valeryl chloride, and optionally for converting said VSN into valsartan, said process being a process which further comprises:
iii) optionally obtaining VSN from said organic and water phases, especially from said organic phase, and
iv) optionally converting the obtained VSN into valsartan.

The carbonate is selected from the group consisting of sodium carbonate, sodium hydrogen carbonate, potassium carbonate, potassium hydrogen carbonate, and mixtures thereof, most preferably the carbonate is sodium carbonate.

A molar ratio between VSV and the carbonate in the flow reactor is from 1:1 to 1:10, preferably from 1:1 to 1:6, most preferably from 1:2 to 1:4.

A molar ratio between VSV and valeryl chloride in the flow reactor is from 1:10 to 10:1, preferably from 1:6 to 6:1, more preferably from 1:3 to 5:1, even more preferably from 1:2 to 4:1.

A solvent for preparing the solution of VSV is water, an aromatic hydrocarbon solvent or a mixture thereof. The aromatic hydrocarbon solvent is preferably selected from the group consisting of toluene, xylene, benzene, ethylbenzene and mixtures thereof, more preferably toluene or xylene, most preferably toluene.

A solvent for preparing the solution of valeryl chloride is an aromatic hydrocarbon solvent preferably selected from the group consisting of toluene, xylene, benzene, ethylbenzene, and mixtures thereof, more preferably toluene or xylene, most preferably toluene.

It is preferable that the same aromatic hydrocarbon solvent is used for preparing the solution of VSV and the solution of valeryl chloride, respectively.

A solvent for preparing a solution of carbonate is water.

The solution of VSV can comprise a solvent which is or comprises water, an aromatic hydrocarbon solvent or a mixture thereof, preferably the aromatic hydrocarbon solvent being selected from the group consisting of toluene, xylene, benzene, ethylbenzene and mixtures thereof, more preferably toluene or xylene, most preferably toluene. The solution of valeryl chloride can comprise a solvent which is or comprises an aromatic hydrocarbon solvent, preferably selected from the group consisting of toluene, xylene, benzene, ethylbenzene, and mixtures thereof, more preferably toluene or xylene, most preferably toluene. The solution of carbonate can comprise a solvent which is or comprises water. Continuous extraction can mean that the extraction takes place in-situ, i.e. at the place where VSN is formed in the flow reactor. Preferably, VSN is extracted into organic phase. Separation of organic and water phases via continuous flow separator can mean that the separation of organic and water phases is carried out in a continuous flow separator.

A volume ratio between aromatic hydrocarbon solvent and water in the flow reactor is from 5:1 to 1:5, preferably from 3:1 to 1:3, more preferably from 2:1 to 1:2, most preferably from 3:2 to 1:1.

A concentration of VSV in the flow reactor is from 0.05 mmol/L to 1 mmol/mL, preferably from 0.05 mmol/mL to 0.5 mmol/mL, more preferably from 0.05 mmol/mL to 0.43 mmol/mL, most preferably from 0.10 mmol/mL to 0.25 mmol/mL.

A concentration of the carbonate in the flow reactor is from 0.05 mmol/L to 5.0 mmol/mL, preferably from 0.05 mmol/mL to 3.0 mmol/mL, more preferably from 0.1 mmol/mL to 2.0 mmol/mL, most preferably from 0.1 mmol/mL to 1.0 mmol/mL.

A concentration of valeryl chloride in the flow reactor is from 0.05 mmol/L to 5.0 mmol/mL, preferably from 0.05 mmol/mL to 3.0 mmol/mL, more preferably from 0.1 mmol/mL to 2.0 mmol/mL, most preferably from 0.1 mmol/mL to 1.0 mmol/mL.

A temperature in the flow reactor is at least 20°C, more preferably at least 40°C, even more preferably at least 45°C, even more preferably the temperature in the flow reactor is between 20°C and 80°C, even more preferably between 40°C and 70°C, even more preferably between 40°C and 60°C, even more preferably between 40°C and 55°C, most preferably between 40°C and 50°C.

Various streams may be added into the flow reactor through distinct or common addition ports. Furthermore, various streams may be added into the flow reactor simultaneously or at distinct addition times.

For example, the first addition stream is added into the flow reactor through the first addition port, the second addition stream is added through the second addition port, and the optional third addition stream is added through the optional third addition port. The flow reactor can be a flow reactor having a first addition port, a second addition port, and optionally an optional third addition port. Optionally, the flow reactor can comprise further optional addition port(s).

Some of reactants and/or reagents may be pre-mixed into common stream prior to addition into the flow reactor.

The first addition stream, the second addition stream, and the optional third addition stream are added into the flow reactor simultaneously. Alternatively, the first addition stream, the second addition stream, and the optional third addition stream are added into the reaction chamber(s) of the flow reactor in successive steps.

A preferred embodiment of the present invention is a process for the preparation of VSN by acylation of VSV with valeryl chloride comprising:
i) providing a first stream containing a solution of VSV and a carbonate;
ii) providing a second stream containing a solution of valeryl chloride;
iii) contacting, preferably continuously mixing, said streams in a flow reactor, thereby producing VSN; and
iv) continuously removing a product stream comprising VSN from said flow reactor, preferably after performing the continuous extraction *in situ,* and then subjecting the product stream comprising VSN to a separation of organic and water phases, preferably via continuous flow separator.

The carbonate is selected from the group consisting of sodium carbonate, sodium hydrogen carbonate, potassium carbonate, potassium hydrogen carbonate, and mixtures thereof, most preferably the carbonate is sodium carbonate.

A solvent for preparing the solution of VSV and the carbonate is water or a mixture of water and an aromatic hydrocarbon solvent, preferably selected from the group consisting of toluene, xylene, benzene, ethylbenzene, and mixtures thereof, more preferably toluene or xylene, most preferably toluene. The aromatic hydrocarbon solvent and water are mixed in a volume ratio from 2:1 to 1:5, preferably from 1:1 to 1:2, most preferably 2:3.

A molar ratio between VSV and the carbonate in the solution of VSV and a carbonate
is from 1:1 to 1:10, preferably from 1:1 to 1:6, most preferably from 1:2 to 1:4.

A concentration of VSV in the solution of VSV and the carbonate is from 0.05 mmol/L to 1 mmol/mL, preferably from 0.1 mmol/mL to 0.5 mmol/mL, more preferably from 0.15 mmol/mL to 0.3 mmol/mL, most preferably from 0.20 mmol/mL to 0.25 mmol/mL.

A concentration of the carbonate in the solution of VSV and the carbonate is from 0.1 mmol/L to 5.0 mmol/mL, preferably from 0.3 mmol/mL to 3.0 mmol/mL, more preferably from 0.4 mmol/mL to 2.0 mmol/mL, most preferably from 0.5 mmol/mL to 1.0 mmol/mL.

A solvent for preparing the solution of valeryl chloride is an aromatic hydrocarbon solvent, preferably selected from the group consisting of toluene, xylene, benzene, ethylbenzene and mixtures thereof, more preferably toluene or xylene, most preferably toluene.

It is preferable that the same aromatic hydrocarbon solvent is used for preparing the solution of VSV and the solution of valeryl chloride, respectively.

A concentration of valeryl chloride in the solution of valeryl chloride is from 0.1 mmol/mL to 5.0 mmol/mL, preferably from 0.5 mmol/mL to 4.0 mmol/mL, more preferably from 0.8 mmol/mL to 2.5 mmol/mL, most preferably from 1.0 mmol/mL to 2.0 mmol/mL.

A molar ratio between VSV and valeryl chloride in the flow reactor is from 1:10 to 10:1, preferably from 1:6 to 6:1, more preferably from 1:4 to 5:1, even more preferably from 1:2 to 4:1.

A molar ratio between VSV and the carbonate in the flow reactor is from 1:1 to 1:10, preferably from 1:1 to 1:6, most preferably from 1:2 to 1:4.

A concentration of VSV in the flow reactor is from 0.05 mmol/L to 1 mmol/mL, preferably from 0.05 mmol/mL to 0.5 mmol/mL, more preferably from 0.05 mmol/mL to 0.43 mmol/mL, most preferably from 0.10 mmol/mL to 0.25 mmol/mL.

A concentration of the carbonate in a flow reactor is from 0.05 mmol/L to 5.0 mmol/mL, preferably from 0.05 mmol/mL to 3.0 mmol/mL, more preferably from 0.1 mmol/mL to 2.0 mmol/mL, most preferably from 0.1 mmol/mL to 1.0 mmol/mL.

A concentration of valeryl chloride in the flow reactor is from 0.05 mmol/L to 5.0 mmol/mL, preferably from 0.05 mmol/mL to 3.0 mmol/mL, more preferably from 0.1 mmol/mL to 2.0 mmol/mL, most preferably from 0.1 mmol/mL to 1.0 mmol/mL.

A volume ratio between aromatic hydrocarbon solvent and water in the flow reactor is from 5:1 to 1:5, preferably from 3:1 to 1:3, more preferably from 2:1 to 1:2, most preferably from 3:2 to 1:1.

In particular, a volume ratio can be determined at a temperature of 25°C, especially at a temperature of 25°C and 1 atm.

Preferably, the first stream containing the solution of VSV and the carbonate is added into the flow reactor through the first addition port and the second stream containing the solution of valeryl chloride is added through the second addition port.

According to a preferred embodiment, the first stream containing the solution of VSV and the carbonate and the second stream containing a solution of valeryl chloride are added into the flow reactor simultaneously.

According to another preferred embodiment, the first stream containing the solution of VSV and the carbonate and the second stream containing the solution of valeryl chloride are added into the flow reactor in successive steps.

The second stream containing the solution of valeryl chloride is added in a flow reactor in one portion or in more portions. If the second stream containing the solution of valeryl chloride is added in more portions it is preferably added in two to five portions, more preferably in two to four portions.

According to another preferred embodiment, the second stream containing the solution of valeryl chloride is added in one portion and a molar ratio between VSV and valeryl chloride in the flow reactor is from 1:1 to 1:5, preferably from 1:1 to 1:4, more preferably from 1:1 to 1:3.

According to another preferred embodiment, the second stream containing the solution of valeryl chloride is added in more portions and a molar ratio between VSV and valeryl chloride in the flow reactor in each individual addition step is from 1:1 to 10:1, preferably from 2:1 to 6:1, more preferably from 2:1 to 5:1.

According to another preferred embodiment, the second stream containing the solution of valeryl chloride is added in more portions and all portions are added into the first flow reactor in successive steps.

According to another preferred embodiment, the second stream containing the solution of valeryl chloride is added in more portions, the first portion is added into the first flow reactor and followed portions are added in successive flow reactors.

According to another preferred embodiment, the second stream containing the solution of valeryl chloride is added in more portions, the first portion is added into the second flow reactor and followed portions are added in successive flow reactors.

A temperature in the flow reactor is at least 20°C, more preferably at least 40°C, even more preferably at least 45°C, even more preferably the temperature in the flow reactor is between 20°C and 80°C, even more preferably between 40°C and 70°C, even more preferably between 40°C and 60°C, even more preferably between 40°C and 55°C, most preferably between 40°C and 50°C.

The solution of VSV and the carbonate is preferably prepared by mixing water, the carbonate, VSV and the aromatic hydrocarbon solvent, preferably toluene, under stirring until all reagents are completely dissolved. Preferably, a mixing is performed at temperature of at least 30°C, more preferably at least 40°C, even more preferably at least 45°C.

Preferably, the stream containing the solution of VSV and the carbonate and the stream comprising valeryl chloride are introduced into the flow reactor at a temperature of at least 30°C, more preferably at least 40°C, even more preferably at least 45°C.

Alternatively, the solution of VSV in the aromatic hydrocarbon solvent, preferably toluene, and the solution of the carbonate in water are prepared separately. VSV solution and the carbonate solution are added into the flow reactor as separated streams that are mixed together in the flow reactor.

Another preferred embodiment of the present invention is a process for the preparation of VSN by acylation of VSV with valeryl chloride comprising:
i) providing a first stream containing a solution of VSV;
ii) providing a second stream containing a solution of carbonate;
iii) providing a third stream containing a solution of valeryl chloride;
iv) contacting, preferably continuously mixing, said streams in a flow reactor, thereby producing VSN; and
v) continuously removing a product stream comprising VSN from said flow reactor, preferably after performing the continuous extraction *in situ,* and then subjecting the product stream comprising VSN to a separation of organic and water phases, preferably via continuous flow separator.

The carbonate is selected from the group consisting of sodium carbonate, sodium hydrogen carbonate, potassium carbonate, potassium hydrogen carbonate, and mixtures thereof, most preferably the carbonate is sodium carbonate.

A solvent for preparing the solution of VSV is water or a mixture of water and an aromatic hydrocarbon solvent, preferably selected from the group consisting of toluene, xylene, benzene, ethylbenzene, and mixtures thereof, more preferably toluene or xylene, most preferably toluene. The aromatic hydrocarbon solvent and water are mixed in a volume ratio from 2:1 to 1:5, preferably from 1:1 to 1:2, most preferably 2:3.

A concentration of VSV in the solution of VSV is from 0.05 mmol/L to 1 mmol/mL, preferably from 0.1 mmol/mL to 0.5 mmol/mL, more preferably from 0.15 mmol/mL to 0.3 mmol/mL, most preferably from 0.20 mmol/mL to 0.25 mmol/mL.

A solvent for preparing the solution of the carbonate is water.

A concentration of the carbonate in the solution of the carbonate is from 0.1 mmol/L to 5.0 mmol/mL, preferably from 0.3 mmol/mL to 3.0 mmol/mL, more preferably from 0.4 mmol/mL to 2.0 mmol/mL, most preferably from 0.5 mmol/mL to 1.0 mmol/mL.

A solvent for preparing the solution of valeryl chloride is an aromatic hydrocarbon solvent, preferably selected from the group consisting of toluene, xylene, benzene, ethylbenzene, and mixtures thereof, more preferably toluene or xylene, most preferably toluene.

It is preferable that the same aromatic hydrocarbon solvent is used for preparing the solution of VSV and the solution of valeryl chloride, respectively.

Preferably, a concentration of valeryl chloride in the solution of valeryl chloride is from 0.1 mmol/mL to 5.0 mmol/mL, preferably from 0.5 mmol/mL to 4.0 mmol/mL, more preferably from 0.8 mmol/mL to 2.5 mmol/mL, most preferably from 1.0 mmol/mL to 2.0 mmol/mL.

A molar ratio between VSV and the carbonate in the flow reactor is from 1:1 to 1:10, preferably from 1:1 to 1:6, most preferably from 1:2 to 1:4.

A molar ratio between VSV and valeryl chloride in the flow reactor is from 1:10 to 10:1, preferably from 1:6 to 6:1, more preferably from 1:3 to 5:1, even more preferably from 1:2 to 4:1.

A concentration of VSV in the flow reactor is from 0.05 mmol/L to 1 mmol/mL, preferably from 0.05 mmol/mL to 0.5 mmol/mL, more preferably from 0.05 mmol/mL to 0.43 mmol/mL, most preferably from 0.10 mmol/mL to 0.25 mmol/mL.

A concentration of the carbonate in the flow reactor is from 0.05 mmol/L to 5.0 mmol/mL, preferably from 0.05 mmol/mL to 3.0 mmol/mL, more preferably from 0.1 mmol/mL to 2.0 mmol/mL, most preferably from 0.1 mmol/mL to 1.0 mmol/mL.

A concentration of valeryl chloride in the flow reactor is from 0.05 mmol/L to 5.0 mmol/mL, preferably from 0.05 mmol/mL to 3.0 mmol/mL, more preferably from 0.1 mmol/mL to 2.0 mmol/mL, most preferably from 0.1 mmol/mL to 1.0 mmol/mL.

A volume ratio between the aromatic hydrocarbon solvent and water in the flow reactor is from 5:1 to 1:5, preferably from 3:1 to 1:3, more preferably from 2:1 to 1:2, most preferably from 3:2 to 1:1.

Preferably, the first stream containing the solution of VSV is added into the flow reactor through the first addition port, the second stream containing the solution of carbonate is added through the second addition port and the third stream containing a solution of valeryl chloride is added through the third addition port. The flow reactor can be a flow reactor having a first addition port, a second addition port, and a third addition port. Optionally, the flow reactor can comprise further optional addition port(s).

According to a preferred embodiment, the first stream containing the solution of VSV, the second stream containing the solution of the carbonate and the third stream containing the solution of valeryl chloride are added into the flow reactor simultaneously.

According to another preferred embodiment, the first stream containing the solution of VSV, the second stream containing the solution of the carbonate and the third stream containing the solution of valeryl chloride are added into the flow reactor in successive steps.

According to another preferred embodiment, the first stream containing the solution of VSV and the second stream containing the solution of the carbonate are added into the flow reactor simultaneously and the third stream containing the solution of valeryl chloride is added into the flow reactor in a successive step.

The third stream containing a solution of valeryl chloride is added into the flow reactor in one portion or in more portions. If the third stream containing the solution of valeryl chloride is added in more portions it is preferably added in two to five portions, preferably in two to four portions.

According to another preferred embodiment, the second stream containing the solution of valeryl chloride is added in one portion and a molar ratio between VSV and valeryl chloride in the flow reactor is from 1:1 to 1:5, preferably from 1:1 to 1:4, more preferably from 1:1 to 1:3.

According to another preferred embodiment, the second stream containing the solution of valeryl chloride is added in more portions and a molar ratio between VSV and valeryl chloride in the flow reactor in each individual step is from 1:1 to 10:1, preferably from 2:1 to 6:1, more preferably from 2:1 to 5:1.

According to another preferred embodiment, the third stream containing the solution of valeryl chloride is added in more portions and all portions are added in the first flow reactor in successive steps.

According to another preferred embodiment, the third stream containing the solution of valeryl chloride is added in more portions, the first portion is added into the first flow reactor and followed portions are added in successive flow reactors.

According to another preferred embodiment, the third stream containing the solution of valeryl chloride is added in more portions, the first portion is added into the second flow reactor and followed portions are added in successive flow reactors.

According to another preferred embodiment, the third stream containing the solution of valeryl chloride is added in more portions and individual portions are added separately in successive flow reactors. Preferably, the first portion is added into the flow reactor simultaneously with the first stream containing the solution of VSV and the second stream containing the solution of the carbonate.

A temperature in the flow reactor is at least 20°C, more preferably at least 40°C, even more preferably at least 45°C, even more preferably the temperature in the flow reactor is between 20°C and 80°C, even more preferably between 40°C and 70°C, even more preferably between 40°C and 60°C, even more preferably between 40°C and 55°C, most preferably between 40°C and 50°C.

According the process of any of embodiments of the present invention continuous extraction takes place in-situ, i.e. at the place where VSN is formed in the flow reactor. Preferably, VSN is extracted into organic phase. After the continuous extraction a separation of organic and water phases can be carried out by any known method. Preferably the separation of organic and water phases is carried out in a continuous flow separator. Preferably, the continuous flow separator is a membrane separator. Preferably, the membrane separator comprises a hydrophobic membrane.

According the process of any of embodiments of the present invention a conversion rate of VSV to VSN is at least 95%, preferably at least 97%, more preferably at least 98%, even more preferably at least 99%, most preferably at least 99.5%.

According to the process of any of embodiments of the present invention a highly pure VSN with a high purity measured by HPLC is obtained. VSN obtained according to a process of the present invention has a purity measured by HPLC of at least 95%, preferably at least 97%, more preferably at least 98% even more preferably at least 99%, most preferably at least 99.5%.

Preferably, VSN is separated and/or isolated from the product stream obtained according to the process of any of embodiments of the present invention before it is further used in a process for preparing valsartan. Preferably, VSN is separated by using a membrane separator. Preferably, the membrane separator comprises a hydrophobic membrane.

The process of the present invention can be performed in any known flow reactor, for example a reactor described in WO 2019/028002. The reactor is desirably of such design that the reaction mixture stream is flowed within a passage shaped to induce mixing throughout the duration of the residence time. Preferably, flow reactors are connected in a such way that the exit of each reactor, except the last reactor, is directly connected to the entrance of the successive reactor to form a flow reactor system. Preferred reactors are reactors G1 Advanced-Flow^{™} reactor from Corning and G4 Advanced-Flow^{™} reactor from Corning.

According to another preferred embodiment the process of the present invention is performed with the flow reactor system comprising several flow reactors connected in the flow reactor system.

According to another preferred embodiment the exit from the last flow reactor of the flow reactor system is connected with the membrane separator. Consequently, the product stream comprising VSN flows continuously from the flow reactor system through membrane separator to obtain organic phase comprising VSN.

Another aspect of the present invention is the process according to the any of the above embodiments comprising further steps of converting obtained VSN to valsartan.

VSN may be converted to valsartan by any known method. For example, process for converting VSN to valsartan are described in Bühlmayer et al., Bioorganic & Medicinal Chemistry Letters, Vol. 4, No. 1, pp. 29-34, 1994, US 7,659,406 and WO 2011/124655.

According to the preferred embodiment the process of converting VSN to valsaratan comprises the following steps:
i) treating VSN with azide, preferably tributyltin azide or a mixture of NaN₃ and ZnCl₂, to form tetrazole intermediate,
ii) hydrolysis of tetrazole intermediate obtained in step i), preferably with hydroxide or carbonate, iii) recovering valsartan.

In particular, the tetrazole intermediate can be N-(1-oxopentyl)-N-[[2'-(1H-tetrazol-5-yl)[1,1'-biphenyl]-4-yl]methyl]-L- valine methyl ester.

Another aspect of the invention is a process for preparing valsartan.

Another embodiment of the present invention is the process for the preparation of valsartan including the process for the preparation of VSN according to any the above embodiments related to the processes for preparing VSN.

Another embodiment of the present invention is a process for the preparation of valsartan comprising the following steps:
i) preparing VSN according to any one of the above embodiments related to the processes for preparing VSN,
ii) treating VSN with azide, preferably tributyltin azide or a mixture of NaN₃ and ZnCl₂, to form tetrazole intermediate,
iii) hydrolysis of tetrazole intermediate obtained in step ii), preferably with hydroxide or carbonate, iv) recovering valsartan.

For example, the hydroxide for hydrolysing tetrazole intermediate can be an alkali hydroxide or an earth alkali hydroxide. For example, the carbonate for hydrolysing tetrazole intermediate can be an alkali carbonate or an earth alkali carbonate.

Another preferred embodiment of the present invention is a process for the preparation of valsartan comprising the following steps:
i-a) contacting, preferably continuously mixing, in a flow reactor a solution of VSV and a solution of valeryl chloride in the presence of a carbonate, thereby producing VSN,
i-b) continuously removing a product stream comprising VSN from said flow reactor, preferably after performing the continuous extraction *in situ,* and then subjecting the product stream comprising VSN to a separation of organic and water phases, preferably via continuous flow separator,
ii) treating VSN with azide, preferably tributyltin azide or a mixture of NaN₃ and ZnCl₂, to form tetrazole intermediate,
iii) hydrolysis of tetrazole intermediate obtained in step ii), preferably with hydroxide or carbonate, iv) recovering valsartan.

For the steps i-a) and i-b) are applicable all embodiments and technical features as described above under the aspect related to the processes for preparing VSN.

Another preferred embodiment of the present invention is a process for the preparation of valsartan comprising the following steps:
i-a) providing a first stream containing a solution of VSV and a carbonate;
i-b) providing a second stream containing a solution of valeryl chloride;
i-c) contacting, preferably continuously mixing, said streams in a flow reactor, thereby producing VSN;and
i-d) continuously removing a product stream comprising VSN from said flow reactor, preferably after performing the continuous extraction *in situ,* and then subjecting the product stream comprising VSN to a separation of organic and water phases, preferably via continuous flow separator,
ii) treating VSN with azide, preferably tributyltin azide or a mixture of NaN₃ and ZnCl₂, to form tetrazole intermediate,
iii) hydrolysis of tetrazole intermediate obtained in step ii), preferably with hydroxide or carbonate, iv) recovering valsartan.

For the steps i-a) to i-d) are applicable all embodiments and technical features as described above under the aspect related to the processes for preparing VSN.

Another preferred embodiment of the present invention is a process for the preparation of valsartan comprising the following steps:
i-a) providing a first stream containing a solution of VSV;
i-b) providing a second stream containing a solution of carbonate;
i-c) providing a third stream containing a solution of valeryl chloride;
i-d) contacting, preferably continuously mixing, said streams in a flow reactor, thereby producing VSN;and
i-e) continuously removing a product stream comprising VSN from said flow reactor, preferably after performing the continuous extraction *in situ,* and then subjecting the product stream comprising VSN to a separation of organic and water phases, preferably via continuous flow separator,
ii) treating VSN with azide, preferably tributyltin azide or a mixture of NaN₃ and ZnCl₂, to form tetrazole intermediate,
iii) hydrolysis of tetrazole intermediate obtained in step ii), preferably with hydroxide or carbonate, iv) recovering valsartan.

For the steps i-a) to i-e) are applicable all embodiments and technical features as described above under the aspect related to the processes for preparing VSN.

### Examples

The following examples are merely illustrative of the present invention and they should not be considered as limiting the scope of the invention in any way. The examples and modifications or other equivalents thereof will become apparent to those versed in the art in the light of the present entire disclosure. If not explictly defined otherwise, the term "room temperature" can have in particular the meaning of 20°C.

### Example 1:

### Preparation of a solution of VSV and a carbonate

18 g of Na₂CO₃, 17 g of VSV and 80 mL of toluene were added to 120 mL of water under stirring at 45 °C until all reagents are dissolved.

### Preparation of a solution a solution of valeryl chloride

1,71 g of valeryl chloride is added to 10 mL of toluene under stirring at room temperature.

### Preparation of VSV

### G1 Advanced-Flow^{™} reactor system was used.

A stream of the solution of VSV and the carbonate was added through the first addition port at a flow speed of 10 mL/min and a stream of the solution of valeryl chloride was added through the second addition port of the first reactor at a flow speed of 0.5 mL/min. The stream of the solution of valeryl chloride was added in four portions.

### Example 2:

### Preparation of a solution of VSV and a carbonate

12.6 g of Na₂CO₃, 17 g of VSV and 80 mL of toluene were added to 120 mL of water under stirring at 45 °C until all reagents are dissolved.

### Preparation of a solution a solution of valeryl chloride

13.7 g of valeryl chloride is added to 80 mL of toluene under stirring at room temperature. Obtained solution is heated to 45 °C.

### Preparation of VSN

### G1 Advanced-Flow^{™} reactor system connected with membrane separator was used.

A stream of the solution of VSV and the carbonate was added through the first addition port at a flow speed of 3 mL/min and a stream of a solution of valeryl chloride was added through the second addition port at a flow speed of 1 mL/min. Both streams were added to the first reactor simultaneously.

### Separation

From the last flow reactor the product stream was pumped through membrane separator with hydrophobic membrane to separate water and organic phase. The organic phase comprising VSN was evaporated to obtain oily product.

### Example 3:

### Preparation of a solution of VSV

5 g of VSV was added to 80 mL of water under stirring at 65 °C until reagent is dissolved.

### Preparation of a solution of a carbonate

5 g of Na₂CO₃ was added to 80 mL of water under stirring at 35 °C until reagent is dissolved.

### Preparation of a solution a solution of valeryl chloride

3.2 mL of valeryl chloride is added to 160 mL of toluene under stirring at room temperature.

### Preparation of VSN

A stream of a solution of VSV was added through the first addition port at a flow speed of 1 mL/min, a stream of a solution of a carbonate was added through the second addition port at a flow speed of 1 mL/min and a stream of a solution of valeryl chloride was added through the third addition port at a flow speed of 2mL/min. All 3 streams were added to the first reactor simultaneously.

### Example 4:

### Preparation of valsartan

To the oily product obtained by the process described in Example 2 was treated with 1-methoxy-2-(2-methoxyethoxy)ethane to remove toluene. 11.3 g of sodium azide and 8.6 g of tin chloride were added under stirring at room temperature. The mixture was heated to 125°C for 22 hours. After cooling the mixture was acidified with sodium nitrite to pH 1 and extracted with tert-butyl methyl ether. The organic phase was washed with water and evaporated to obtain crude valsartan. Crude valsartan was dissolved in ethyl acetate at a higher temperature. Then the solution was filtered and cooled to 0°C and stirred for 2h. Pure valsartan was filtered off and dried under reduced pressure.

## Claims

1. A process for the preparation of (S)-methyl 2-(N-((2'-cyano-[1,1'-biphenyl]-4-yl)methyl)pentanamido)-3-methylbutanoate comprising:
i) contacting, preferably continuously mixing, in a flow reactor a solution of (S)-methyl 2-(((2'-cyano-[1,1'-biphenyl]-4-yl)methyl)amino)-3-methylbutanoate hydrochloride and a solution of valeryl chloride in the presence of a carbonate, thereby producing (S)-methyl 2-(N-((2'-cyano-[1,1'-biphenyl]-4-yl)methyl)pentanamido)-3-methylbutanoate; and
ii) continuously removing a product stream comprising (S)-methyl 2-(N-((2'-cyano-[1,1'-biphenyl]-4-yl)methyl)pentanamido)-3-methylbutanoate from said flow reactor, preferably after performing the continuous extraction *in situ,* and then subjecting the removed product stream comprising (S)-methyl 2-(N-((2'-cyano-[1,1'-biphenyl]-4-yl)methyl)pentanamido)-3-methylbutanoate to a separation of organic and water phases, preferably via continuous flow separator, wherein
- a molar ratio between (S)-methyl 2-(((2'-cyano-[1,1'-biphenyl]-4-yl)methyl)amino)-3-methylbutanoate hydrochloride and the carbonate in the flow reactor is from 1:1 to 1:10, preferably from 1:1 to 1:6, most preferably from 1:2 to 1:4,
- a molar ratio between (S)-methyl 2-(((2'-cyano-[1,1'-biphenyl]-4-yl)methyl)amino)-3-methylbutanoate hydrochloride and valeryl chloride in the flow reactor is from 1:10 to 10:1, preferably from 1:6 to 8:1, more preferably from 1:3 to 6:1, most preferably from 1:2 to 4:1, and
- a solvent present in the flow reactor is or comprises a mixture of water and an aromatic hydrocarbon solvent, preferably the aromatic hydrocarbon solvent being selected from the group consisting of toluene, xylene, benzene, ethylbenzene and mixtures thereof.

2. The process according to claim 1, wherein
- a concentration of (S)-methyl 2-(((2'-cyano-[1,1'-biphenyl]-4-yl)methyl)amino)-3-methylbutanoate hydrochloride in the flow reactor is from 0.05 mmol/L to 1 mmol/mL, preferably from 0.05 mmol/mL to 0.5 mmol/mL, more preferably from 0.05 mmol/mL to 0.43 mmol/mL, most preferably from 0.10 mmol/mL to 0.25 mmol/mL,
- a concentration of the carbonate in the flow reactor is from 0.05 mmol/L to 5.0 mmol/mL, preferably from 0.05 mmol/mL to 3.0 mmol/mL, more preferably from 0.1 mmol/mL to 2.0 mmol/mL, most preferably from 0.1 mmol/mL to 1.0 mmol/mL, and
- a concentration of valeryl chloride in the flow reactor is from 0.05 mmol/L to 5.0 mmol/mL, preferably from 0.05 mmol/mL to 3.0 mmol/mL, more preferably from 0.1 mmol/mL to 2.0 mmol/mL, most preferably from 0.1 mmol/mL to 1.0 mmol/mL.

3. The process according to claim 1 or claim 2, wherein step i) is or comprises:
a-i) providing a first stream containing a solution of (S)-methyl 2-(((2'-cyano-[1,1'-biphenyl]-4-yl)methyl)amino)-3-methylbutanoate hydrochloride and a carbonate;
a-ii) providing a second stream containing a solution of valeryl chloride;
a-iii) contacting, preferably continuously mixing, said streams in a flow reactor, thereby producing (S)-methyl 2-(N-((2'-cyano-[1,1'-biphenyl]-4-yl)methyl)pentanamido)-3-methylbutanoate.

4. The process according claim 1, wherein a solvent for preparing the solution of (S)-methyl 2-(((2'-cyano-[1,1'-biphenyl]-4-yl)methyl)amino)-3-methylbutanoate hydrochloride and the carbonate is water or a mixture of water and an aromatic hydrocarbon solvent, preferably selected from the group consisting of toluene, xylene, benzene, ethylbenzene and mixtures thereof.

5. The process according to claim 3 or claim 4, wherein
- a molar ratio between (S)-methyl 2-(((2'-cyano-[1,1'-biphenyl]-4-yl)methyl)amino)-3-methylbutanoate hydrochloride and the carbonate in the solution of (S)-methyl 2-(((2'-cyano-[1,1'-biphenyl]-4-yl)methyl)amino)-3-methylbutanoate hydrochloride and a carbonate is from 1:1 to 1:10, preferably from 1:1 to 1:6, most preferably from 1:2 to 1:4,
- a concentration of (S)-methyl 2-(((2'-cyano-[1,1'-biphenyl]-4-yl)methyl)amino)-3-methylbutanoate hydrochloride in the solution of (S)-methyl 2-(((2'-cyano-[1,1'-biphenyl]-4-yl)methyl)amino)-3-methylbutanoate hydrochloride and the carbonate is from 0.05 mmol/L to 1 mmol/mL, preferably from 0.1 mmol/mL to 0.5 mmol/mL, more preferably from 0.15 mmol/mL to 0.3 mmol/mL, most preferably from 0.20 mmol/mL to 0.25 mmol/mL, and
- a concentration of the carbonate in the solution of (S)-methyl 2-(((2'-cyano-[1,1'-biphenyl]-4-yl)methyl)amino)-3-methylbutanoate hydrochloride and the carbonate is from 0.1 mmol/L to 5.0 mmol/mL, preferably from 0.3 mmol/mL to 3.0 mmol/mL, more preferably from 0.4 mmol/mL to 2.0 mmol/mL, most preferably from 0.5 mmol/mL to 1.0 mmol/mL.

6. The process according to any one of claims 3 to 5, wherein a solvent for preparing the solution of valeryl chloride is an aromatic hydrocarbon solvent, preferably selected from the group consisting of toluene, xylene, benzene, ethylbenzene and mixtures thereof.

7. The process according to any one of claims 3 to 6, wherein a concentration of valeryl chloride in the solution of valeryl chloride is from 0.1 mmol/mL to 5.0 mmol/mL, preferably from 0.5 mmol/mL to 4.0 mmol/mL, more preferably from 0.8 mmol/mL to 2.5 mmol/mL, most preferably from 1.0 mmol/mL to 2.0 mmol/mL.

8. The process according to claim 1 or claim 2, wherein step i) is or comprises:
a-i) providing a first stream containing a solution of (S)-methyl 2-(((2'-cyano-[1,1'-biphenyl]-4-yl)methyl)amino)-3-methylbutanoate hydrochloride;
a-ii) providing a second stream containing a solution of carbonate;
a-iii) providing a third stream containing a solution of valeryl chloride;
a-iv) contacting, preferably continuously mixing, said streams in a flow reactor, thereby producing (S)-methyl 2-(N-((2'-cyano-[1,1'-biphenyl]-4-yl)methyl)pentanamido)-3-methylbutanoate.

9. The process according to claim 8, wherein
- a solvent for preparing a solution of (S)-methyl 2-(((2'-cyano-[1,1'-biphenyl]-4-yl)methyl)amino)-3-methylbutanoate hydrochloride is water or a mixture of water and an aromatic hydrocarbon solvent, preferably selected from the group consisting of toluene, xylene, benzene, ethylbenzene and mixtures thereof,
- a solvent for preparing the solution of carbonate is water, and
- a solvent for preparing the solution of valeryl chloride is an aromatic hydrocarbon solvent, preferably selected from the group consisting of toluene, xylene, benzene, ethylbenzene and mixtures thereof.

10. The process according to claim 8 or claim 9, wherein
- a concentration of (S)-methyl 2-(((2'-cyano-[1,1'-biphenyl]-4-yl)methyl)amino)-3-methylbutanoate hydrochloride in the solution of (S)-methyl 2-(((2'-cyano-[1,1'-biphenyl]-4-yl)methyl)amino)-3-methylbutanoate hydrochloride is from 0.05 mmol/L to 1 mmol/mL, preferably from 0.1 mmol/mL to 0.5 mmol/mL, more preferably from 0.15 mmol/mL to 0.3 mmol/mL, most preferably from 0.20 mmol/mL to 0.25 mmol/mL,
- a concentration of the carbonate in the solution of the carbonate is from 0.1 mmol/L to 5.0 mmol/mL, preferably from 0.3 mmol/mL to 3.0 mmol/mL, more preferably from 0.4 mmol/mL to 2.0 mmol/mL, most preferably from 0.5 mmol/mL to 1.0 mmol/mL, and
- a concentration of valeryl chloride in the solution of valeryl chloride is from 0.1 mmol/mL to 5.0 mmol/mL, preferably from 0.5 mmol/mL to 4.0 mmol/mL, more preferably from 0.8 mmol/mL to 2.5 mmol/mL, most preferably from 1.0 mmol/mL to 2.0 mmol/mL.

11. The process according to any one of claims 1 to 10 comprising further step(s) of converting obtained (S)-methyl 2-(N-((2'-cyano-[1,1'-biphenyl]-4-yl)methyl)pentanamido)-3-methylbutanoate to valsartan.

12. The process according to claim 11 comprising the following further steps:
i) treating (S)-methyl 2-(N-((2'-cyano-[1,1'-biphenyl]-4-yl)methyl)pentanamido)-3-methylbutanoate with azide, preferably tributyltin azide or a mixture of NaN₃ and ZnCl₂, to form tetrazole intermediate,
ii) hydrolysis of tetrazole intermediate obtained in step i), preferably with hydroxide or carbonate,
iii) recovering valsartan.

13. A process for the preparation of valsartan including the process for the preparation of (S)-methyl 2-(N-((2'-cyano-[1,1'-biphenyl]-4-yl)methyl)pentanamido)-3-methylbutanoate according to any one of claims 1 to 10.

14. A process for the preparation of valsartan comprising the following steps:
i) preparing (S)-methyl 2-(N-((2'-cyano-[1,1'-biphenyl]-4-yl)methyl)pentanamido)-3-methylbutanoate according to any one of claims 1 to 10,
ii) treating (S)-methyl 2-(N-((2'-cyano-[1,1'-biphenyl]-4-yl)methyl)pentanamido)-3-methylbutanoate with azide, preferably tributyltin azide or a mixture of NaN₃ and ZnCl₂, to form tetrazole intermediate,
iii) hydrolysis of tetrazole intermediate obtained in step ii), preferably with hydroxide or carbonate,
iv) recovering valsartan.
